# EUROPÄISCHE PATENTSCHRIFT

(11) **EP 3 405 451 B1**
(45) Veröffentlichungstag und Bekanntmachung des Hinweises auf die Patenterteilung: **01.01.2020**
(21) Anmeldenummer: 17701642.5
(22) Anmeldetag: 12.01.2017
(51) Int. Cl.: C07C 209/78, C07C 211/50

(54) **VERFAHREN ZUR HERSTELLUNG EINES AROMATISCHEN POLYAMINGEMISCHES**
METHOD FOR PRODUCING AN AROMATIC POLYAMINE MIXTURE
PROCÉDÉ DE PRODUCTION D'UN MÉLANGE DE POLYAMINES AROMATIQUE

(30) Priorität: 20.01.2016 EP 16152074
(43) Veröffentlichungstag der Anmeldung: 28.11.2018
(73) Patentinhaber: BASF SE, 67056 Ludwigshafen am Rhein (DE)
(72) Erfinder: LADNAK, Viktor, 67056 Ludwigshafen (DE); TAURO, Silvia, 67056 Ludwigshafen (DE); HEINEN, Kerstin, 67056 Ludwighafen (DE); THIELE, Kai, 2040 Antwerpen (BE); METZNER, Klaus-Peter, DECEASED (DE); MATTKE, Torsten, 67056 Ludwigshafen (DE)
(74) Vertreter: BASF IP Association
(86) Internationale Anmeldenummer: PCT/EP2017/050517
(87) Internationale Veröffentlichungsnummer: WO 2017/125302

(56) Entgegenhaltungen:
- EP-A1- 1 167 343
- DE-A1- 19 804 915
- US-A- 3 860 637

## Beschreibung

Die Erfindung betrifft ein Verfahren zur Herstellung eines aromatischen Polyamingemisches, das im wesentlichen 4,4'-Methylen(diphenylamin) und höhere Homologe von Methylen(diphenylamin) enthält.

Die Herstellung von Methylen(diphenylamin) (= MDA) erfolgt üblicherweise durch kontinuierliche oder diskontinuierliche Umsetzung von Anilin mit Formaldehyd in Gegenwart von sauren Katalysatoren. Bei dieser Umsetzung entsteht ein Gemisch aus überwiegend 4,4'-MDA, 2,4'-MDA, 2,2'-MDA und höheren Homologen von MDA, das als "Roh-MDA" bezeichnet wird. Die Isomeren 2,4'-MDA und 2,2'-MDA sind jedoch oft unerwünscht, da bei einer Folgeumsetzung mit Phosgen die Verbindungen 2,4'- und 2,2'-Methylen-(diphenylisocyanat) (= MDI) gebildet werden. Diese Verbindungen weisen ortho-ständige Isocyanatgruppen auf, die bei einer Urethanisierungsreaktion mit Polyolen nur unvollständig abreagieren.

Zur Verringerung des Gehalts an 2,4'-MDA und 2,2'-MDA im Roh-MDA sind verschiedene Verfahren bekannt.

US-A 2009/0240077 offenbart ein Verfahren zur Herstellung von Di- und Polyaminen der Diphenylmethanreihe durch Umsetzung von Anilin und Formaldehyd in Gegenwart eines sauren Katalysators, wobei das eingesetzte Anilin weniger als 0,5 Gew.-% an carbonylgruppenhaltigen Verbindungen enthält. Aus der organischen Phase des erhaltenen Reaktionsprodukts wird überschüssiges Anilin destillativ entfernt und zumindest teilweise wieder in die Reaktion zurückgeführt. Die erhaltenen Di- und Polyamine weisen verbesserte Farbwerte auf. Nachteilig ist jedoch, dass das Verfahren wenig selektiv und somit der Gehalt an 4,4'-MDA und seinen höheren Homologen verbesserungsbedürftig ist.

US 3,860,637 beschreibt ein Verfahren zur Herstellung von Polyaminen, insbesondere von 4,4'-MDA, durch Umsetzung von Anilin mit Formaldehyd zu einem Rohproduktgemisch, wobei ein Strom von 2,2'-MDA und 2,4'-MDA abgetrennt und in den Anilin-Formaldehyd-Strom rückgeführt wird. Die Abtrennung der 2,2'- und 2,4'-Isomere erfolgt durch eine Vakuumdestillation unter nicht näher ausgeführten Bedingungen.

EP-A-1167343 beschreibt ein Verfahren zur Herstellung eines aromatischen Polyamingemisches, das 4,4'-Methylen(diphenylamin) und höhere Homologe von Methylen(diphenylamin) enthält, wobei in Stufe I in einer Anlage, die eine Mischzone (a), eine Kondensationszone (b) und eine Umlagerungszone (c) umfasst, Anilin mit Formaldehyd in Gegenwart eines Katalysators zu einem Rohproduktgemisch umgesetzt wird. Anilin und Wasser werden von dem Rohproduktgemisch abgetrennt und danach wird von dem so erhaltenen anilinarmen Rohproduktgemisch ein Gemisch aus 2,2'-Methylendi(phenylamin) und 2,4'-Methylendi(phenylamin) destillativ abgetrennt. Die Abtrennung des Gemisches aus 2,2'-MDA und 2,4'-MDA erfolgt mittels einer Destillationskolonne, die eine theoretische Trennstufenzahl von mindestens 40 aufweist, bei einer Temperatur von 180 bis 280°C, einem Kopfdruck von 0,1 bis 10 mbar und einem Sumpfdruck von 8 bis 20 mbar.

Das eingesetzte Formaldehyd wird in mindestens zwei Teile aufteilt, wovon ein Teil der Kondensationszone (b) und ein Teil mit dem abgetrennten Gemisch aus 2,2'-Methylendi(phenylamin) und 2,4'-Methylendiphenylamin vermischt und der Umlagerungszone c) der Stufe I zugeführt wird.

Nachteilig an den beiden vorgenannten Verfahren ist, dass die destillative Trennung von 4,4-MDA und 2,4-MDA schwierig ist und zu hohen Energie- und Investitionskosten führt.

Ein weiterer Nachteil ist, dass im erhaltenen Roh-MDA niedrigsiedende Verunreinigungen enthalten sind, die nach der Destillation des Roh-MDA zusammen mit dem Gemisch aus 2,2-MDA und 2,4-MDA in die Reaktion mit Formaldehyd zurückgeführt werden. Diese Nebenkomponenten können sich im Rezyklierstrom ansammeln (bei Nichtreaktivität gegenüber Formaldehyd) oder (bei Reaktivität gegenüber Formaldehyd) zu neuen Verunreinigungen sowie zu erhöhten Konzentrationen an Verunreinigungen im MDI, z.B. einer erhöhten Konzentration an N-Methyl-4,4'-methylendianilin (N-Methyl-MDA) führen. Diese niedrigsiedenden Verunreinigungen bewirken oftmals, dass die aus dem Zielprodukt nach der Umsetzung mit Phosgen erhaltenen Folgeprodukte von polymerem MDI hohe Chlorwerte und eine dunkle oder gelbliche Verfärbung aufweisen.

Aufgabe der vorliegenden Erfindung ist es daher, ein verbessertes, insbesondere energiekostensenkendes, Verfahren zur Herstellung eines aromatischen Polyamingemisches durch Umsetzung von Anilin mit Formaldehyd bereitzustellen, welches im wesentlichen 4,4'-Methylen(diphenylamin) und höhere Homologe von Methylen(diphenylamin) enthält und das eine möglichst hohe Selektivität von 4,4'-MDA bezogen auf 2-Kern-MDA aufweist. Darüber hinaus soll durch das Verfahren der Einsatz von Rohmaterialien wie Salzsäure bzw. Natronlauge deutlich reduziert werden. Eine weitere Aufgabe ist es, dass das durch das Verfahren erhaltene Produkt weitgehend frei von Nebenkomponenten, insbesondere N-Methyl-MDA, sein soll und die aus dem Zielprodukt erhaltenen Folgeprodukte von polymerem MDI verbesserte Farbwerte als auch Chlorwerte aufweisen sollen.

Im Sinne der Erfindung sind unter 2-Kern-MDA 2,2'-MDA, 2,4'-MDA und 4,4'-MDA zu verstehen. Die Selektivität von 4,4'-MDA bezogen auf 2-Kern-MDA ist definiert wie folgt:
Selektivität (4,4'-MDA) = n (4,4'-MDA) / [n (2,2'-MDA) + n (2,4'-MDA) + n (4,4'-MDA)], wobei n die jeweilige molare Menge darstellt.

Die Aufgabe konnte durch das erfindungsgemäße Verfahren gemäß den Ansprüchen gelöst werden.

Gegenstand der Erfindung ist daher ein Verfahren zur Herstellung eines aromatischen Polyamingemisches, das 4,4'-Methylen(diphenylamin) und höhere Homologe von Methylen(diphenylamin) enthält, durch
(i) Umsetzung von Anilin mit Formaldehyd mittels eines sauren Katalysators zu einem Rohproduktgemisch (I), wobei (i) in einer Anlage durchgeführt wird, die eine Mischzone (a), eine Kondensationszone (b), eine Umlagerungszone (c) und gegebenenfalls eine Nachreaktions zone (d) umfasst;
(ii) Neutralisation des nach (i) erhaltenen Rohproduktgemischs (I) und Abtrennung der entstehenden Salze;
(iii) Abtrennung von Anilin von dem nach (ii) erhaltenen Rohproduktgemisch und gegebenenfalls Rückführung des Anilins in die Mischzone (a); danach
(iv) Destillation des nach (iii) erhaltenen Rohproduktgemisches unter Abtrennung
   (iv-1) eines Gemisches (II) bestehend aus Methylendi(phenylamin)-Isomeren (II-1) und davon verschiedenen Nebenkomponenten (II-2), wobei, bezogen auf (II-1), der Anteil an 4,4'-Methylendi(phenylamin) 8 bis 20 Gew.-% beträgt, und, bezogen auf (II), der Anteil an Nebenkomponenten (II-2) maximal 0,3 Gew.-% beträgt, und
   (iv-2) eines Leichtsiedergemisches bestehend aus Nebenkomponenten (II-2) und Methylendi(phenylamin)-Isomeren (II-1), in welchem der Anteil der Nebenkomponenten (II-2) mindestens 55 Gew.-% beträgt; und
(v) Rückführung des Gemisches (II) in eine der Zonen (a) bis (d), bevorzugt (a).

Das durch das erfindungsgemäße Verfahren erhaltene Polyamingemisch enthält im wesentlichen 4,4'-Methylen(diphenylamin) und höhere Homologe von Methylen(diphenylamin). Darunter ist im Rahmen dieser Erfindung zu verstehen, dass das Polyamingemisch im Allgemeinen einen Gehalt an 4,4'-MDA und höheren Homologen von MDA von grösser 85 Gew.-%, bevorzugt von grösser 90 Gew.-%, besonders bevorzugt von grösser 94 Gew.-%, ganz besonders bevorzugt von grösser 95 Gew.-% aufweist. Der Gehalt an 2,2'-MDA und 2,4'-MDA ist im Allgemeinen kleiner als 10 Gew.-%, bevorzugt kleiner als 6 Gew.-% und besonders bevorzugt kleiner als 5 Gew.-%.

Unter höheren Homologen sind erfindungsgemäß drei- bis zehnkernige, insbesondere drei- bis vierkernige, Homologe von Methylen(diphenylamin) zu verstehen. Im Sinne der Erfindung werden die höheren Homologe, falls nicht explizit bezeichnet, auch als polymeres MDA (pMDA) zusammengefasst.

Das erfindungsgemäß in Schritt (iv-1) abgetrennte und in Schritt (v) zurückgeführte Gemisch (II) besteht aus Methylendi(phenylamin)-Isomeren (II-1) und davon verschiedenen Nebenkomponenten (II-2). Der Anteil an Nebenkomponenten (II-2) beträgt maximal 0,3 Gew.-%, bezogen auf das Gemisch (II).

Bevorzugt beträgt in dem Gemisch (II) (= Rückführ- oder Rezyklierstrom) der Anteil an Nebenkomponenten (II-2) maximal 0,1 Gew.-%, besonders bevorzugt maximal 0,01 Gew.-%. Die Summe der Komponenten (II-1) und (II-2) in dem Gemisch (II) beträgt 100 Gew.-%.

Bei den Methylendi(phenylamin)-Isomeren (II-1) handelt es sich insbesondere um ein Gemisch aus 2,2'-, 2,4' und 4,4'-Methylendi(phenylamin), worin der Anteil an 4,4'-Methylendi(phenylamin) 8 bis 20 Gew.-%, bevorzugt 9 bis 16 Gew.-%, besonders bevorzugt 10 bis 14 Gew.-%, beträgt. In dem Methylendi(phenylamin)-Isomer-Gemisch (II-1) addieren sich die Anteile der verschiedenen Methylendi(phenylamin)-Isomere zu 100 Gew.-%.

Die Nebenkomponenten (II-2) enthalten keine Methylendi(phenylamin)-Isomere (II-1). Bei den Nebenkomponenten (II-2), handelt es sich im Allgemeinen um Verbindungen, die bei Normaldruck eine Siedetemperatur (Ts) gleich oder größer der Siedetemperatur von Anilin und kleiner der Siedetemperatur von 2,2-MDA aufweisen. Oftmals, aber nicht beschränkend, handelt es sich dabei um Anilin, Aminobenzylanilin, Methylchinolin, Formanilid, Methylencarboxanilid, Diphenylamin, 2-Aminobiphenyl, N(para-tolyl)phenlyamin, 1,2,3,4-Tetrahydroacridin, Acridinsäure, 9,10-Dihydroacridin, N-Methyl-4,4'-methylendianilin sowie deren Isomere, insbesondere um Anilin, Diphenylamin, Acridinsäure und Dihydroacridine.

Das erfindungsgemäß in Schritt (iv-2) abgetrennte Leichtsiedergemisch besteht aus mindestens 55 Gew.-%, bevorzugt mindestens 80 Gew.-%, besonders bevorzugt mindestens 90 Gew.-% Nebenkomponenten (II-2) und Methylendi(phenylamin)-Isomeren (II-1), welche jeweils wie vorstehend definiert sind, und wobei die Summe der Komponenten (II-2) und (II-I) in dem Leichtsiedergemisch 100 Gew.-% beträgt.

In der vorliegenden Erfindung erfolgt die Umsetzung (i) von Anilin mit Formaldehyd zu einem Rohproduktgemisch (I) in einer Anlage mit den Reaktionszonen (a) bis (c) und gegebenenfalls (d) wie nachstehend beschrieben:
(i-a) Vermischen von Anilin oder gegebenenfalls eines Gemisches aus Anilin und Katalysator mit Formaldehyd in einer geeigneten Mischvorrichtung. Diese Reaktionszone wird als Mischzone (a) bezeichnet.
(i-b) Reaktion in einer Reaktionsvorrichtung zumeist in einem Temperaturbereich von 20 bis 100°C, bevorzugt von 30 bis 60°C. Hierbei erfolgt überwiegend eine Kondensation der Edukte zu sogenannten Aminobenzylaminen (ABAs), die als Zwischenprodukt auftreten. Diese Reaktionszone wird im Rahmen dieser Erfindung als Kondensationszone (b) bezeichnet.
(i-c) Reaktion in einer Reaktionsvorrichtung zumeist in einem Temperaturbereich von 60 bis 140 °C, bevorzugt von 70 bis 100 °C. Hierbei erfolgt überwiegend eine Umlagerung der als Zwischenprodukt gebildeten Aminobenzylamine zu Roh-MDA. Diese Zone wird im Rahmen dieser Erfindung als Umlagerungszone (c) bezeichnet.
(i-d) Reaktion in einer Reaktionsvorrichtung zumeist in einem Temperaturbereich von 70 bis 180 °C, bevorzugt von 80 bis 120 °C. Hierbei erfolgt überwiegend eine Vervollständigung der vorstehend erwähnten Umlagerungsreaktion zu Roh-MDA. Diese Reaktionszone wird im Rahmen dieser Erfindung als Nachreaktionszone (d) bezeichnet. Stufe (i-d) ist optional.

Als Mischvorrichtungen für die Reaktionszone (a) sind beispielsweise Mischpumpen, Düsen und statische als auch dynamische Mischer geeignet.

Als Reaktionsvorrichtung für die Reaktionszonen (b) bis (d) sind beispielsweise Rohrreaktoren, Rührreaktoren und Reaktionskolonnen geeignet. Ferner können die Reaktionszonen (b) bis (d) der Stufe (i) in einem einzigen Reaktor, der gegebenenfalls unterschiedliche Temperaturbereiche aufweist, durchgeführt werden. Dies ist beispielsweise in einer Bodenkolonne möglich.

Das Rohproduktgemisch aus der Reaktionszone (c) oder gegebenenfalls (d) wird üblicherweise in eine Aufarbeitungszone (e) überführt. Dort erfolgt die (ii) Neutralisation des nach (i) erhaltenen Rohproduktgemischs (I) und die Abtrennung der entstehenden Salze. Die Neutralisation des Rohproduktgemischs (I) erfolgt alkalisch, bevorzugt mit wässriger NaOH. Die dabei entstehenden Salze können in der Wasserphase abgetrennt werden.

Danach wird (iii) Anilin von dem nach (ii) erhaltenen Rohproduktgemisch abgetrennt und gegebenenfalls in die Mischzone (a) zurückgeführt. Die Abtrennung des Anilins erfolgt mit Hilfe üblicher Methoden, insbesondere destillativ (z. B. Rotationsverdampfer, Destillationskolonne etc.), wobei die Bedingungen so gewählt werden, dass Anilin in einer Reinheit von mindestens 95% erhalten wird. Vorzugsweise erfolgt die Abtrennung des Anilins mit Hilfe von einer oder mehreren, vorzugsweise hintereinander geschalteten, Destillationskolonnen. Für die Anilindestillation hat sich ein Druck von 1 bis 9 mbar, vorzugsweise 3 bis 8 mbar, besonders bevorzugt 4 bis 6 mbar, bei einer Temperatur von 27 bis 110°C, vorzugsweise 43 bis 100°C, besonders bevorzugt 47 bis 89°C, gemessen in der letzten Kolonne, am Kopf derer das Anilin gewonnen wird, als vorteilhaft erwiesen. Unter den vorgenannten Bedingungen lässt sich Anilin in einer Reinheit von mindestens 95 %, bevorzugt >97%, ganz besonders bevorzugt >98% erhalten.

Bevorzugt wird das abgetrennte Anilin rezykliert und in die Mischzone (a) zurückgeführt.

Danach erfolgt (iv) die Destillation des erhaltenen anilinarmen Rohproduktgemisches unter Abtrennung
(iv-1) eines Gemisches (II) bestehend aus Methylendi(phenylamin)-Isomeren (II-1) und davon verschiedenen Nebenkomponenten (II-2), wobei, bezogen auf (II-1), der Anteil an 4,4'-Methylendi(phenylamin) 8 bis 20 Gew.-% beträgt, und, bezogen auf (II), der Anteil an Nebenkomponenten (II-2) maximal 0,3 Gew.-% beträgt, und
(iv-2) eines Leichtsiedergemisches bestehend aus Nebenkomponenten (II-2) und Methylendi(phenylamin)-Isomeren (II-1), in welchem der Anteil der Nebenkomponenten (II-2) mindestens 55 Gew.-% beträgt.

Bevorzugt erfolgt in Schritt (iv) die Abtrennung (iv-2) des Leichtsiedergemisches gleichzeitig mit der Abtrennung (iv-1) des Gemisches (II) in einer Kolonne mit Trennwand oder Seitenstromabzug.

Weiterhin bevorzugt erfolgt in Schritt (iv) die Abtrennung (iv-2) des Leichtsiedergemisches aus dem Kopfstrom einer ersten Destillationskolonne, der erhaltene Sumpfstrom wird einer weiteren (zweiten) nacheinander angeordneten Destillationskolonne zugeführt und (iv-1) das Gemisch (II) aus dem Kopfstrom der weiteren Destillationskolonne abgetrennt.

Erfindungsgemäß besonders bevorzugt ist die gleichzeitige Abtrennung des Leichtsiedergemisches und des Gemisches (II) mit Hilfe mindestens einer Trennwand- oder Seitenstromkolonne, insbesondere einer Trennwandkolonne. Dabei werden das Leichtsiedergemisch aus dem Kopfstrom und das Gemisch (II) aus dem Seitenstrom der Trennwand- oder der Seitenstromkolonne abgetrennt.

Das Leichtsiedergemisch wird nicht in den Rezyklierstrom zurückgeführt und entweder aus dem erfindungsgemäßen Prozess ausgeschleust oder mit dem nach Schritt (iv) erhaltenen MDA-Produktgemisch vermischt. Letzteres führt zu einer Maximierung der Ausbeute.

Bevorzugt wird das Leichtsiedergemisch aus dem Prozess ausgeschleust. Das hat den Vorteil, dass Zielprodukte mit verbesserter Qualität, d.h. insbesondere einem geringeren N-Methyl-4,4'-methylendianilin-Gehalt, erhalten werden.

Gemäß einer ersten bevorzugten Ausführungsform wird zur Abtrennung (iv-2) der Leichtsieder die Destillation in einer (ersten) üblichen Destillationskolonne mit einer theoretischen Trennstufenzahl von 2 bis 15, bevorzugt von 6 bis 10 durchgeführt. Die Abtrennung (iv-1) des Gemisches (II) erfolgt in einer weiteren (zweiten) Destillationskolonne mit einer theoretischen Trennstufenzahl von 10 bis 20, bevorzugt von 13 bis 16. Die Destillation in der ersten Destillationskolonne wird üblicherweise bei einem Kopfdruck von 1 bis 10 mbar, bevorzugt 1 bis 3 mbar, und bei einer Temperatur von 120 bis 210°C, bevorzugt von 135 bis 177°C, besonders bevorzugt 147 bis 166°C durchgeführt. Danach wird der erhaltene Sumpfstrom zur Abtrennung (iv-1) des Gemisches (II) in eine weitere (zweite) Destillationskolonne wie zuvor beschrieben überführt und bei einem Kopfdruck von 1 bis 10 mbar, bevorzugt 4 bis 6 mbar, und bei einer Temperatur von 170 bis 230°C, bevorzugt 200 bis 215°C eine Destillation durchgeführt.

Bevorzugt wird für die Destillation in Schritt (iv) eine Trennwand- oder Seitenstromkolonne, die im oberen Bereich zwischen Seitenstrom und Kopfstrom eine theoretische Trennstufenzahl von 2 bis 15, bevorzugt von 6 bis 10 aufweist, und im unteren Bereich zwischen Sumpf und Seitenstrom eine theoretische Trennstufenzahl von 10 bis 20, bevorzugt von 13 bis 16 aufweist, eingesetzt.

Gemäß einer zweiten bevorzugten Ausführungsform wird eine wie vorstehend beschriebene Trennwand- oder Seitenstromkolonne verwendet. Gemäß dieser Ausführungsform wird die Destillation in Schritt (iv) bei einem Kopfdruck von 1 bis 10 mbar, bevorzugt 1 bis 3 mbar, und einer Temperatur von 120 bis 210°C, bevorzugt 135 bis 177°C, besonders bevorzugt 147 bis 166°C zur Abtrennung (iv-2) der Leichtsieder und bei einem Seitenstromdruck von 1 bis 10 mbar, bevorzugt 4 bis 6 mbar, und bei einer Temperatur von 170 bis 230°C, bevorzugt 200 bis 215°C zur Abtrennung (iv-1) des Gemisches (II) durchgeführt. Dabei werden die Leichtsieder als Kopfstrom und das Gemisch (II) als Seitenstrom abgetrennt.

Besonders bevorzugt sind Kolonnen mit Einbauten wie z.B. Packungen Sulzer BXplus.

Das abgetrennte Gemisch (II) wird dann gemäß Schritt (v) in eine der Zonen (a) bis (d), bevorzugt in Zone (a) zurückgeführt.

Darunter ist auch zu verstehen, dass das abgetrennte Gemisch (II) nicht direkt zurückgeführt wird, sondern in dafür geeigneten Vorrichtungen zwischengelagert werden kann. Ferner ist es beispielsweise auch möglich, das nach dem erfindungsgemäßen Verfahren abgetrennte Gemisch (II) einer Zone (a) bis (d), bevorzugt einer Zone (a) einer weiteren Anlage zuzuführen oder ein aus einer weiteren Anlage abgetrenntes Gemisch (II) in eine der Zonen (a) bis (d), bevorzugt (a), der erfindungsgemäß verwendeten Anlage zuzuführen.

Das nach dem erfindungsgemäßen Verfahren erhältliche Polyamingemisch, welches im wesentlichen 4,4'-Methylen(diphenylamin) und höhere Homologe von Methylen(diphenylamin) enthält, kann üblicherweise als Sumpfstrom der Kolonne in Schritt (iv) abgetrennt werden.

Danach kann das erhaltene Polyamingemisch einer Phosgenierung zugeführt werden.

Das nach dem erfindungsgemäßen Verfahren erhaltene Polyamingemisch kann auch mit dem abgetrennten Leichtsiedergemisch wieder zusammengeführt werden und gemeinsam einer optionalen Phosgenierung zugeführt werden.

Bevorzugt wird das Leichtsiedergemisch aus dem Verfahren ausgeschleust und das nach dem erfindungsgemäßen Verfahren erhaltene Polyamingemisch allein zur optionalen Phosgenierung geführt.

Bei dem erfindungsgemäßen Verfahren wird mindestens ein saurer Katalysator eingesetzt. Bevorzugt wird ein saurer Katalysator verwendet. Handelt es sich dabei um einen homogenen Katalysator, so wird dieser bevorzugt im Gemisch mit Anilin zugegeben. Als homogene Katalysatoren sind Mineralsäuren, besonders Salzsäure, Schwefelsäure und Phosphorsäure, besonders bevorzugt Salzsäure, geeignet. Wird als homogener Katalysator Chlorwasserstoff verwendet, so kann dieser gasförmig oder als wässrige Lösung eingesetzt werden.

Handelt es sich um einen Heterogenkatalysator, so wird dieser zumeist in die Reaktionsvorrichtungen der Zonen (b) bis (d) der Stufe (i) eingebracht.

Als heterogene Katalysatoren sind Ionenaustauscher, Tone, Zeolithe, Silica-Alumina-, Wolfram- oder Molybdänoxide auf diversen Trägern wie beispielsweise TiO₂, ZrO₂, HfO₂, SnO₂ und Al₂O₃ vorteilhaft und saure Ionenaustauscher besonders vorteilhaft. Die genannten Katalysatoren können gegebenenfalls in beliebiger Kombination eingesetzt werden.

Alternativ ist es auch möglich, Anilin in Form eines sauren Salzes in das erfindungsgemäße Verfahren einzubringen. Bevorzugt liegt dabei das Amin in Form eines Hydrochlorids, Sulfats oder Phosphats vor.

Wird ein homogener Katalysator verwendet, so liegt zumeist ein molares Verhältnis Anilin : Katalysator von 1 : 0,6 bis 1 : 0,001, bevorzugt von 1 : 0,3 bis 1: 0,05 vor. Im Fall der heterogenen Katalyse liegt der Katalysator bezogen auf Anilin mit 1 bis 99 Gew.-%, bevorzugt 20 bis 60 Gew.-% vor. Es ist auch möglich, ein Gemisch aus homogenem und heterogenem Katalysator zu verwenden.

Das erfindungsgemäße Verfahren kann ferner in Gegenwart eines Lösungsmittels durchgeführt werden. Besonders geeignet sind Ether, gegebenenfalls mit Halogenatomen substituierte aromatische und aliphatische Lösungsmittel, Wasser und Gemische davon. Beispiele hierfür sind Benzol, Toluol, Monochlorbenzol, Dichlorbenzol, Dimethylformamid (DMF), Tetrahydrofuran (THF) und Diethylisophthalat (DEIP). Besonders bevorzugt wird Monochlorbenzol verwendet. Bevorzugt wird Anilin selbst als Lösungsmittel eingesetzt.

Formaldehyd kann dem erfindungsgemäßen Verfahren in Form von monomerem Formaldehyd und/oder in Form von höheren Homologen, sogenannten Poly(oxymethylen)glykolen, zugeführt werden.

Das molare Verhältnis Anilin : Formaldehyd beträgt im allgemeinen 1,75 bis 10 : 1, bevorzugt 2 bis 4 : 1.

Das erfindungsgemäße Verfahren wird bevorzugt kontinuierlich durchgeführt.

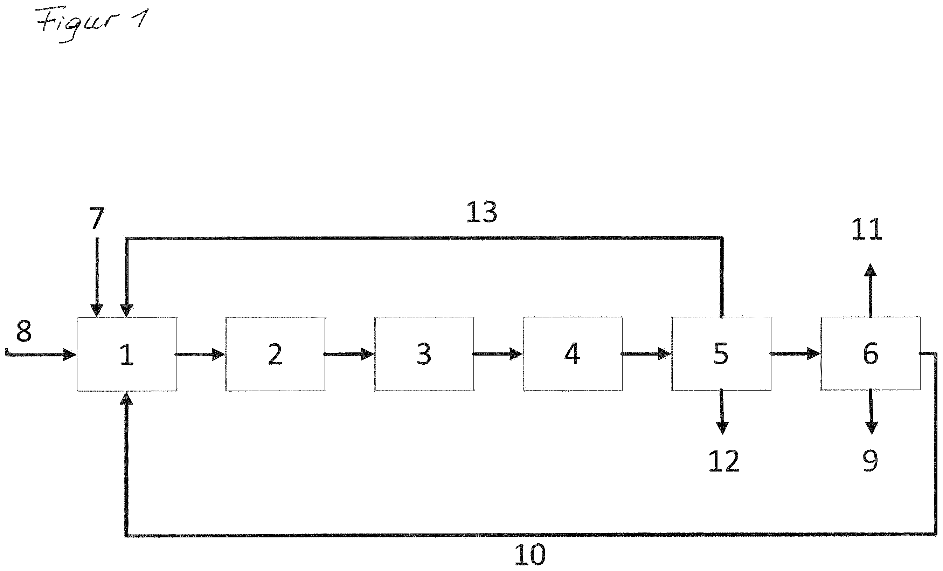

Figur 1 veranschaulicht eine bevorzugte Ausführungsform des erfindungsgemäßen Verfahrens.

In der Figur bedeutet:
- 1: Mischzone (a)
- 2: Kondensationszone (b)
- 3: Umlagerungszone (c)
- 4: Nachreaktionszone (d)
- 5: Aufarbeitungszone (e)
- 6: Destillationskolonne(n)
- 7: Zufuhr von Anilin im Gemisch mit Katalysator
- 8: Zufuhr von Formaldehyd
- 9: Austrag von 4,4'-MDA und höheren Homologen von MDA
- 10: Rückführung des Gemisches (II) aus 2,2'-MDA, 2,4'-MDA und 4,4'-MDA
- 11: Abtrennung Leichtsieder
- 12: Austrag von Wasser und Salzen
- 13: Rückführung von Anilin

Gemäß Figur 1 wird das abgetrennte Gemisch (II) enthaltend 2,2'-MDA, 2,4'-MDA und 4,4'-MDA rückgeführt 10 und mit Anilin 7, dem ein saurer Katalysator beigemischt ist, und Formaldehyd 8 in einer Mischzone (a) 1 vermischt. Bevorzugt wird hier eine Mischkammer einer Reaktionsmischpumpe oder eine Düse, die der Kondensationszone (b) 2 vorgeschaltet sind, verwendet, so dass möglichst rasch eine vollständige Durchmischung eintritt. Eine rasche Durchmischung vermindert dabei unerwünschte Parallelreaktionen.

Ein Rohr- oder Rührreaktor stellt hierbei beispielsweise eine Reaktionszone dar, die eine Kondensations- (b) 2, Umlagerungs- (c) 3 und Nachreaktionszone (d) 4 umfasst. Üblicherweise wird Anilin 7 oder Anilinhydrochlorid vorgelegt und eine Formalinlösung 8 zudosiert. Die Zugabe kann direkt in den Kessel oder über eine geeignete Mischeinheit erfolgen. Im Allgemeinen wird anschließend ein geeignetes Temperaturprofil angelegt. Nach erfolgter Kondensations- und Umlagerungsreaktion erfolgt Entleeren des Reaktors und eine anschließende Aufarbeitung des Rohprodukts. In einer Aufarbeitungszone (e) 5 erfolgen die Neutralisation des Rohprodukts und der Austrag von Wasser und Salzen 12, sowie die Anilinabtrennung. Das abgetrennte Anilin wird in die Mischzone (a) 1 zurückgeführt 13. Danach wird das Rohprodukt einer Destillation in einer Destillationskolonne 6 zugeführt. Dabei werden die Leichtsieder 11 abgetrennt. Das abgetrennte Gemisch (II) aus 2,2'-MDA, 2,4'-MDA und 4,4'-MDA wird in die Mischzone (a) 1 zurückgeführt 10 und das Produkt von 4,4'-MDA und höheren Homologen von MDA wird ausgetragen 9.

Die vorliegende Erfindung soll anhand der nachfolgenden Beispiele veranschaulicht werden.

### Beispiele

### Nicht erfindungsgemäßes Beispiel 1:

Rückführung eines 2,2'-/2,4'- und 4,4'-MDA-Isomerengemisches, erhalten durch Abtrennung mittels einer Destillationskolonne mit einer theoretischen Stufenzahl von 40, in die Mischzone (a) einer MDA Anlage gemäß Figur 1 ohne vorherige Abtrennung eines Leichtsiederstromes enthaltend gebildete Nebenkomponenten 625 g Anilin (Reinheit >99%) und 19 g eines Rückführstromes gemäß Tab. 2 wurden in einem Rührbehälter vorgelegt und 131 g einer 32 %igen Salzsäure hinzugegeben. Über eine Mischpumpe wurden kontinuierlich 213 g/h einer 35,5 %igen Formalinlösung bei 60°C über 1 h zugemischt. Die Mischpumpe befindet sich im Umpumpkreislauf des Mischbehälters. Der Umpumpkreislauf betrug 25 l/h. Nach 1 h wurde die Formalinzudosierung gestoppt und das Reaktionsgemisch auf 90°C aufgeheizt und für 1,5 h weiter umgepumpt. Anschließend wurde der Umpumpkreislauf gestoppt und das Reaktionsgemisch auf 120°C erwärmt und für 2h weiter dispergiert.

Die ausreagierte Reaktionsmischung wurde nach Abkühlung auf 80°C mit wässriger Natronlauge (25 Gew-%) neutralisiert. Nach Abtrennung der wässrigen Phase wird die organische Phase mit Wasser gewaschen und anschließend das Anilin in einem Rotationsverdampfer bei 1 mbar und einer Ölbadtemperatur von 100°C abdestilliert.

Tabelle 1 zeigt die Produktzusammensetzung nach der Aufarbeitungszone (e):

**Tabelle 1**

| | |
|---|---|
| 2,2-MDA Gew.% | 0,6 |
| 2,4-MDA Gew.% | 9,4 |
| 4,4-MDA Gew.% | 59,1 |
| 3-Kern-MDA Gew.% | 20,3 |
| 4-Kern-MDA Gew.% | 5,6 |
| N-Methyl-MDA Gew. % | 0,120 |

Der Anteil an Nebenkomponenten mit einem Siedepunkt gleich oder größer Anilin und kleiner 2,2'-MDA in der Produktmischung gemäß Tab. 1 betrug etwa 500 ppm.

Zur Abtrennung des 2,2'-/2,4'- und 4,4'-MDA-Rückführstromes wurde die Produktmischung gemäß Tab. 1 auf Stufe 20 in eine Destillationskolonne mit einer theoretischen Stufenzahl von 40 zugeführt. Bei einem Kopfdruck von 10 mbar und einer Kopftemperatur von 224°C wurde bei einem Rücklaufverhältnis von 9 g/g ein Rückführstrom mit folgender Zusammensetzung am Kopf der Kolonne erhalten:

**Tabelle 2**

| | |
|---|---|
| 2,4'-MDA | 73,5% |
| 2,2'-MDA | 11,5% |
| 4,4'-MDA | 13,9% |
| Summe Nebenkomponenten | 1,1% |

Die Zusammensetzung dieses Rückführstromes entspricht der Zusammensetzung des Rückführstromes, der zu Beginn des Versuches in die Mischzone (a) hinzugegeben wurde.

Die im Sumpf der Kolonne resultierende Produktmischung hat folgende Produktzusammensetzung:

**Tabelle 3**

| | |
|---|---|
| 2,2-MDA Gew.% | 0,07 |
| 2,4-MDA Gew.% | 6,5 |
| 4,4-MDA Gew.% | 61,2 |
| 3-Kern-MDA Gew.% | 21,3 |
| pMDA Gew.% | 10,8 |
| N-Methyl-MDA Gew. % | 0,125 |

### Erfindungsgemäßes Beispiel 1:

Rückführung eines 2,2'-/2,4'- und 4,4'-MDA-Isomerengemisches, erhalten durch Abtrennung mittels einer ersten und einer zweiten Destillationskolonne mit einer theoretischen Stufenzahl von 8 bzw. 15, in die Mischzone (a) einer MDA Anlage gemäß Figur 1 mit vorheriger Abtrennung eines Leichtsiederstromes, enthaltend gebildete Nebenkomponenten, und Ausschleusung des Leichtsiederstromes aus dem Prozess
720 g Anilin (Reinheit >99%) und 19 g eines Rückführstromes gemäß Tab. 5 wurden in einem Rührbehälter vorgelegt und 154 g einer 32 %igen Salzsäure hinzugegeben. Über eine Mischpumpe wurden kontinuierlich 253 g/h einer 35,5 %igen Formalinlösung bei 60°C über 1 h zugemischt. Die Mischpumpe befindet sich im Umpumpkreislauf des Mischbehälters. Der Umpumpkreislauf betrug 25 l/h. Nach 1 h wurde die Formalinzudosierung gestoppt und das Reaktionsgemisch auf 90°C aufgeheizt und für 1,5 h weiter umgepumpt. Anschließend wurde der Umpumpkreislauf gestoppt und das Reaktionsgemisch auf 120°C erwärmt und für 2h weiter dispergiert.

Die ausreagierte Reaktionsmischung wurde nach Abkühlung auf 80°C mit wässriger Natronlauge (25 Gew-%) neutralisiert. Nach Abtrennung der wässrigen Phase wird die organische Phase mit Wasser gewaschen und anschließend das Anilin in einem Rotationsverdampfer bei 1mbar und einer Ölbadtemperatur von 100°C abdestilliert.

Tabelle 4 zeigt die Produktzusammensetzung nach der Aufarbeitungszone (e):

**Tabelle 4**

| | |
|---|---|
| 2,2-MDA Gew.% | 0,4 |
| 2,4-MDA Gew.% | 8,8 |
| 4,4-MDA Gew.% | 59,5 |
| 3-Kern-MDA Gew.% | 21,4 |
| 4-Kern-MDA Gew.% | 6,6 |
| N-Methyl-MDA Gew. % | 0,096 |

Der Anteil an Nebenkomponenten mit einem Siedepunkt gleich oder größer Anilin und kleiner 2,2'-MDA in der Produktmischung gemäß Tab. 4 betrug etwa 500 ppm.

Zur Abtrennung des 2,2'-/2,4'- und 4,4'-MDA-Rückführstromes wurde die Produktmischung gemäß Tab. 4 auf Stufe 5 in eine erste Destillationskolonne mit einer theoretischen Stufenzahl von 8 zugeführt. Bei einem Kopfdruck von 2mbar und einer Kopftemperatur von 152°C wurde bei einem Rücklaufverhältnis von 150g/g ein Leichtsiederstrom mit einem Gehalt an Nebenkomponenten von 86% erhalten und aus dem Prozess ausgeschleust.

Der im Sumpf der ersten Kolonne resultierende Produktstrom wird auf Stufe 10 in eine zweite Destillationskolonne mit einer theoretischen Trennstufenzahl von 15 zugeführt. Bei einem Kopfdruck von 5mbar und einer Kopftemperatur von 207°C wird bei einem Rücklaufverhältnis von 26g/g ein Rückführstrom mit folgender Zusammensetzung am Kopf der Kolonne erhalten:

**Tabelle 5**

| | |
|---|---|
| 2,4'-MDA | 75,5% |
| 2,2'-MDA | 11,8% |
| 4,4'-MDA | 12,6% |
| Summe Nebenkomponenten | 0,1% |

Die Zusammensetzung dieses Rückführstromes entspricht der Zusammensetzung des Rückführstromes, der zu Beginn des Versuches in die Mischzone (a) hinzugegeben wurde.

Die im Sumpf der zweiten Kolonne resultierende Produktmischung hat folgende Produktzusammensetzung:

**Tabelle 6**

| | |
|---|---|
| 2,2-MDA Gew.% | <0,01 |
| 2,4-MDA Gew.% | 6,3 |
| 4,4-MDA Gew.% | 61,2 |
| 3-Kern-MDA Gew.% | 22,2 |
| p-MDA Gew.% | 10,2 |
| N-Methyl-MDA Gew. % | 0,100 |

Der Gehalt an N-Methyl-MDA konnte von 0,125 % auf 0,100 % gesenkt werden. Dies entspricht einer Abnahme der unerwünschten Nebenkomponenten um 20%.

### Erfindungsgemäßes Beispiel 2:

Rückführung eines 2,2'-/2,4'- und 4,4'-MDA-Isomerengemisches, erhalten durch Abtrennung mittels einer ersten und einer zweiten Destillationskolonne mit einer theoretischen Stufenzahl von 10 bzw. 15, in die Mischzone (a) einer MDA Anlage gemäß Figur 1 mit vorheriger Abtrennung eines Leichtsiederstromes, enthaltend gebildete Nebenkomponenten, und Rückführung des Leichtsiederstromes in den Produktstrom der zweiten Destillationskolonne
690 g Anilin (Reinheit >99%) und 19 g eines Rückführstromes gemäß Tab. 8 wurden in einem Rührbehälter vorgelegt und 144 g einer 32 %igen Salzsäure hinzugegeben. Über eine Mischpumpe wurden kontinuierlich 235 g/h einer 35,5 %igen Formalinlösung bei 60°C über 1 h zugemischt. Die Mischpumpe befindet sich im Umpumpkreislauf des Mischbehälters. Der Umpumpkreislauf betrug 25 l/h. Nach 1 h wurde die Formalinzudosierung gestoppt und das Reaktionsgemisch auf 90°C aufgeheizt und für 1,5 h weiter umgepumpt. Anschließend wurde der Umpumpkreislauf gestoppt und das Reaktionsgemisch auf 120°C erwärmt und für 2h weiter dispergiert.

Die ausreagierte Reaktionsmischung wurde nach Abkühlung auf 80°C mit wässriger Natronlauge (25 Gew-%) neutralisiert. Nach Abtrennung der wässrigen Phase wird die organische Phase mit Wasser gewaschen und anschließend das Anilin in einem Rotationsverdampfer bei 1mbar und einer Ölbadtemperatur von 100°C abdestilliert.

Tabelle 7 zeigt die Produktzusammensetzung nach der Aufarbeitungszone (e):

**Tabelle 7**

| | |
|---|---|
| 2,2-MDA Gew.% | 0,5 |
| 2,4-MDA Gew.% | 9,0 |
| 4,4-MDA Gew.% | 58,8 |
| 3K-MDA Gew.% | 21,2 |
| 4K-MDA Gew.% | 6,5 |
| N-methyl-MDA Gew. % | 0,086 |

Der Anteil an Nebenkomponenten mit einem Siedepunkt gleich oder größer Anilin und kleiner 2,2'-MDA in der Produktmischung gemäß Tab. 7 betrug etwa 500 ppm.

Zur Abtrennung des Rückführstromes wurde die Produktmischung gemäß Tab. 7 auf Stufe 4 in eine erste Destillationskolonne mit einer theoretischen Stufenzahl von 10 zugeführt. Bei einem Kopfdruck von 2 mbar und einer Kopftemperatur von 150°C wurde bei einem Rücklaufverhältnis von 500g/g ein Leichtsiederstrom mit einem Gehalt an Nebenkomponenten von 82% erhalten.

Der im Sumpf der ersten Kolonne resultierende Produktstrom wird auf Stufe 10 in eine zweite Destillationskolonne mit einer theoretischen Trennstufenzahl von 15 zugeführt. Bei einem Kopfdruck von 4 mbar und einer Kopftemperatur von 202°C wird bei einem Rücklaufverhältnis von 22 g/g ein Rückführstrom mit folgender Zusammensetzung am Kopf der Kolonne erhalten:

**Tabelle 8**

| | |
|---|---|
| 2,4'-MDA | 75,0% |
| 2,2'-MDA | 12,4% |
| 4,4'-MDA | 12,6% |
| Summe Nebenkomponenten | <0,01% |

Die Zusammensetzung dieses Rückführstromes entspricht der Zusammensetzung des Rückführstromes, der zu Beginn des Versuches in die Mischzone (a) hinzugegeben wurde.

Die im Sumpf der zweiten Kolonne resultierende Produktmischung wurde mit dem Kopfstrom der ersten Kolonne (Leichtsiederstrom) vermischt. Die daraus resultierende finale Produktmischung hat folgende Produktzusammensetzung:

**Tabelle 9**

| | |
|---|---|
| 2,2-MDA Gew.% | 0,02 |
| 2,4-MDA Gew.% | 6,3 |
| 4,4-MDA Gew.% | 60,6 |
| 3K-MDA Gew.% | 22,0 |
| p-MDA Gew.% | 10,9 |
| N-methyl-MDA Gew. % | 0,09 |

Der Gehalt an N-Methyl-MDA konnte von 0,125 % auf 0,09 % gesenkt werden. Dies entspricht einer Abnahme der unerwünschten Nebenkomponente um 28%.

## Patentansprüche

1. Verfahren zur Herstellung eines aromatischen Polyamingemisches, das 4,4'-Methylen(diphenylamin) und höhere Homologe von Methylen(diphenylamin) enthält, durch
(i) Umsetzung von Anilin mit Formaldehyd mittels eines sauren Katalysators zu einem Rohproduktgemisch (I), wobei (i) in einer Anlage durchgeführt wird, die eine Mischzone (a), eine Kondensationszone (b), eine Umlagerungszone (c) und gegebenenfalls eine Nachreaktionszone (d) umfasst;
(ii) Neutralisation des nach (i) erhaltenen Rohproduktgemischs (I) und Abtrennung der entstehenden Salze;
(iii) Abtrennung von Anilin von dem nach (ii) erhaltenen Rohproduktgemisch und gegebenenfalls Rückführung des Anilins in die Mischzone (a); danach
(iv) Destillation des nach (iii) erhaltenen Rohproduktgemisches unter Abtrennung
(iv-1) eines Gemisches (II) bestehend aus Methylendi(phenylamin)-Isomeren (II-1) und davon verschiedenen Nebenkomponenten (II-2), wobei, bezogen auf (II-1), der Anteil an 4,4'-Methylendi(phenylamin) 8 bis 20 Gew.-% beträgt, und, bezogen auf (II), der Anteil an Nebenkomponenten (II-2) maximal 0,3 Gew.-% beträgt, und
(iv-2) eines Leichtsiedergemisches bestehend aus Nebenkomponenten (II-2) und Methylendi(phenylamin)-Isomeren (II-1), in welchem der Anteil der Nebenkomponenten (II-2) mindestens 55 Gew.-% beträgt; und
(v) Rückführung des Gemisches (II) in eine der Zonen (a) bis (d).

2. Verfahren gemäß Anspruch 1, **dadurch gekennzeichnet, dass** in Schritt (iv) die Abtrennung (iv-2) des Leichtsiedergemisches gleichzeitig mit der Abtrennung (iv-1) des Gemisches (II) in einer Kolonne mit Trennwand oder Seitenstromabzug erfolgt.

3. Verfahren gemäß Anspruch 1, **dadurch gekennzeichnet, dass** in Schritt (iv) die Abtrennung (iv-2) des Leichtsiedergemisches aus dem Kopfstrom einer ersten Destillationskolonne erfolgt, der erhaltene Sumpfstrom einer weiteren nacheinander angeordneten Destillationskolonne zugeführt wird und (iv-1) das Gemisch (II) aus dem Kopfstrom der weiteren Destillationskolonne abgetrennt wird.

4. Verfahren gemäß Anspruch 1 oder 2, **dadurch gekennzeichnet, dass** zur Destillation in Schritt (iv) eine Trennwand- oder Seitenstromkolonne, die im oberen Bereich zwischen Seitenstrom und Kopfstrom eine theoretische Trennstufenzahl von 2 bis 15, bevorzugt von 6 bis 10 aufweist, und im unteren Bereich zwischen Sumpf und Seitenstrom eine theoretische Trennstufenzahl von 10 bis 20, bevorzugt von 13 bis 16 aufweist, eingesetzt wird.

5. Verfahren gemäß Anspruch 1 oder 3, **dadurch gekennzeichnet, dass** zur Abtrennung (iv-2) des Leichtsiedergemisches die Destillation in einer ersten Destillationskolonne mit einer theoretischen Trennstufenzahl von 2 bis 15, bevorzugt von 6 bis 10 durchgeführt wird und zur Abtrennung (iv-1) des Gemisches (II) in einer weiteren Destillationskolonne mit einer theoretischen Trennstufenzahl von 10 bis 20, bevorzugt von 13 bis 16 durchgeführt wird.

6. Verfahren gemäß Anspruch 4, **dadurch gekennzeichnet, dass** die Destillation in Schritt (iv) bei einem Kopfdruck von 1 bis 10 mbar, bevorzugt 1 bis 3 mbar, und einer Temperatur von 120 bis 210°C, bevorzugt 135 bis 177°C zur Abtrennung (iv-2) des Leichtsiedergemisches und bei einem Seitenstromdruck von 1 bis 10 mbar, bevorzugt 4 bis 6 mbar, und bei einer Temperatur von 170 bis 230°C, bevorzugt 200 bis 215°C zur Abtrennung (iv-1) des Gemisches (II) durchgeführt wird.

7. Verfahren gemäß Anspruch 5, **dadurch gekennzeichnet, dass** die Destillation in der ersten Destillationskolonne bei einem Kopfdruck von 1 bis 10 mbar, bevorzugt 1 bis 3 mbar, und bei einer Temperatur von 120 bis 210°C, bevorzugt von 135 bis 177°C und in der weiteren Destillationskolonne bei einem Kopfdruck von 1 bis 10 mbar, bevorzugt 4 bis 6 mbar, und bei einer Temperatur von 170 bis 230°C, bevorzugt 200 bis 215°C durchgeführt wird.

8. Verfahren nach einem der Ansprüche 1 bis 7, **dadurch gekennzeichnet, dass** das Leichtsiedergemisch aus dem Prozess ausgeschleust wird.

9. Verfahren nach einem der Ansprüche 1 bis 8, **dadurch gekennzeichnet, dass** das Gemisch (II) in die Zone (a) zurückgeführt wird.

## Claims

1. A process for preparing an aromatic polyamine mixture comprising 4,4'-methylenedi(phenylamine) and higher homologues of methylenedi(phenylamine) by
(i) reaction of aniline with formaldehyde by means of an acid catalyst to form a crude product mixture (I), where (i) is carried out in a plant which comprises a mixing zone (a), a condensation zone (b), a rearrangement zone (c) and optionally an after-reaction zone (d);
(ii) neutralization of the crude product mixture (I) obtained in (i) and removal of the salts formed;
(iii) separation of aniline from the crude product mixture obtained in (ii) and optionally recirculation of the aniline to the mixing zone (a); then
(iv) distillation of the crude product mixture obtained in (iii) so as to separate off
(iv-1) a mixture (II) consisting of methylene-di(phenylamine) isomers (II-1) and secondary components (II-2) which are different therefrom, where, based on (II-1), the proportion of 4,4'-methylenedi(phenylamine) is from 8 to 20% by weight and, based on (II), the proportion of secondary components (II-2) is not more than 0.3% by weight, and
(iv-2) a low boiler mixture which consists of secondary components (II-2) and methylene-di(phenylamine) isomers (II-1) and in which the proportion of the secondary components (II-2) is at least 55% by weight; and
(v) recirculation of the mixture (II) to one of the zones (a) to (d).

2. The process according to claim 1, wherein, in step (iv), the isolation (iv-2) of the low boiler mixture is carried out simultaneously with the isolation (iv-1) of the mixture (II) in a column having a dividing wall or a side stream offtake.

3. The process according to claim 1, wherein, in step (iv), the isolation (iv-2) of the low boiler mixture is effected from the overhead stream from a first distillation column, the bottom stream obtained is fed to a further successive distillation column and (iv-1) the mixture (II) is separated off from the overhead stream from the further distillation column.

4. The process according to claim 1 or 2, wherein a dividing wall or side stream column which has from 2 to 15, preferably from 6 to 10, theoretical plates in the upper region between the side stream and the overhead stream and from 10 to 20, preferably from 13 to 16, theoretical plates in the lower region between the bottom and the side stream is used for the distillation in step (iv).

5. The process according to claim 1 or 3, wherein the distillation is carried out in a first distillation column having from 2 to 15, preferably from 6 to 10, theoretical plates for the isolation (iv-2) of the low boiler mixture and is carried out in a further distillation column having from 10 to 20, preferably from 13 to 16, theoretical plates for the isolation (iv-1) of the mixture (II).

6. The process according to claim 4, wherein the distillation in step (iv) is carried out at a pressure at the top of from 1 to 10 mbar, preferably from 1 to 3 mbar, and a temperature of from 120 to 210°C, preferably from 135 to 177°C, for separating off (iv-2) the low boiler mixture and at a side stream pressure of from 1 to 10 mbar, preferably from 4 to 6 mbar, and at a temperature of from 170 to 230°C, preferably from 200 to 215°C, for separating off (iv-1) the mixture (II).

7. The process according to claim 5, wherein the distillation in the first distillation column is carried out at a pressure at the top of from 1 to 10 mbar, preferably from 1 to 3 mbar, and at a temperature of from 120 to 210°C, preferably from 135 to 177°C, and in the further distillation column at a pressure at the top of from 1 to 10 mbar, preferably from 4 to 6 mbar, and at a temperature of from 170 to 230°C, preferably from 200 to 215°C.

8. The process according to any of claims 1 to 7, wherein the low boiler mixture is discharged from the process.

9. The process according to any of claims 1 to 8, wherein the mixture (II) is recirculated to the zone (a).

## Revendications

1. Procédé de fabrication d'un mélange de polyamines aromatiques, qui contient de la 4,4'-méthylène(diphénylamine) et des homologues supérieurs de la méthylène(diphénylamine), par :
(i) la mise en réaction d'aniline avec du formaldéhyde au moyen d'un catalyseur acide pour former un mélange de produits bruts (I), (i) étant réalisé dans une unité qui comprend une zone de mélange (a), une zone de condensation (b), une zone de réarrangement (c) et éventuellement une zone de réaction secondaire (d) ;
(ii) la neutralisation du mélange de produits bruts (I) obtenu selon (i) et la séparation des sels formés ;
(iii) la séparation de l'aniline du mélange de produits bruts obtenu selon (ii) et éventuellement le recyclage de l'aniline dans la zone de mélange (a) ; puis
(iv) la distillation du mélange de produits bruts obtenu selon (iii) avec séparation de (iv-1) un mélange (II) constitué par des isomères de méthylène-di(phénylamine) (II-1) et des composants secondaires (II-2) différents de ceux-ci, la proportion de 4,4'-méthylène-di(phénylamine) par rapport à (II-1) étant de 8 à 20 % en poids, et la proportion de composants secondaires (II-2) par rapport à (II) étant d'au plus 0,3 % en poids, et (iv-2) un mélange de composants de point d'ébullition faible constitué par des composants secondaires (II-2) et des isomères de méthylène-di(phénylamine) (II-1), dans lequel la proportion des composants secondaires (II-2) est d'au moins 55 % en poids ; et
(v) le recyclage du mélange (II) dans une des zones (a) à (d).

2. Procédé selon la revendication 1, **caractérisé en ce qu'**à l'étape (iv), la séparation (iv-2) du mélange de composants de point d'ébullition faible a lieu simultanément avec la séparation (iv-1) du mélange (II) dans une colonne munie d'une paroi de séparation ou d'un soutirage de courant latéral.

3. Procédé selon la revendication 1, **caractérisé en ce qu'**à l'étape (iv), la séparation (iv-2) du mélange de composants de point d'ébullition faible a lieu à partir du courant de tête d'une première colonne de distillation, le courant de fond obtenu est introduit dans une colonne de distillation supplémentaire agencée en aval et (iv-1) le mélange (II) est séparé à partir du courant de tête de la colonne de distillation supplémentaire.

4. Procédé selon la revendication 1 ou 2, **caractérisé en ce que**, pour la distillation à l'étape (iv), une colonne à paroi de séparation ou à courant latéral, qui comprend dans la zone supérieure entre le courant latéral et le courant de tête un nombre d'étapes de séparation théoriques de 2 à 15, de préférence de 6 à 10, et qui comprend dans la zone inférieure entre le fond et le courant latéral un nombre d'étapes de séparation théoriques de 10 à 20, de préférence de 13 à 16, est utilisée.

5. Procédé selon la revendication 1 ou 3, **caractérisé en ce que**, pour la séparation (iv-2) du mélange de composants de point d'ébullition faible, la distillation est réalisée dans une première colonne de distillation contenant un nombre d'étapes de séparation théoriques de 2 à 15, de préférence de 6 à 10, et, pour la séparation (iv-1) du mélange (II), est réalisée dans une colonne de distillation supplémentaire contenant un nombre d'étapes de séparation théoriques de 10 à 20, de préférence de 13 à 16.

6. Procédé selon la revendication 4, **caractérisé en ce que** la distillation à l'étape (iv) est réalisée à une pression de tête de 1 à 10 mbar, de préférence de 1 à 3 mbar, et à une température de 120 à 210 °C, de préférence de 135 à 177 °C, pour la séparation (iv-2) du mélange de composants de point d'ébullition faible, et à une pression de courant latéral de 1 à 10 mbar, de préférence de 4 à 6 mbar, et à une température de 170 à 230 °C, de préférence de 200 à 215 °C, pour la séparation (iv-1) du mélange (II).

7. Procédé selon la revendication 5, **caractérisé en ce que** la distillation dans la première colonne de distillation est réalisée à une pression de tête de 1 à 10 mbar, de préférence de 1 à 3 mbar, et à une température de 120 à 210 °C, de préférence de 135 à 177 °C, et, dans la colonne de distillation supplémentaire, à une pression de tête de 1 à 10 mbar, de préférence de 4 à 6 mbar, et à une température de 170 à 230 °C, de préférence de 200 à 215 °C.

8. Procédé selon l'une quelconque des revendications 1 à 7, **caractérisé en ce que** le mélange de composants de point d'ébullition bas est évacué du processus.

9. Procédé selon l'une quelconque des revendications 1 à 8, **caractérisé en ce que** le mélange (II) est recyclé dans la zone (a).
